# EUROPEAN PATENT APPLICATION

(11) **EP 1 672 064 A1**
(43) Date of publication of application: **21.06.2006**
(21) Application number: 04773482.7
(22) Date of filing: 22.09.2004
(51) Int. Cl.: C12N 15/09, C12N 1/19, C12N 1/21, C12N 5/10

(54) **EFFICIENT METHOD OF PREPARING DNA INVERTED REPEAT STRUCTURE**

(30) Priority: 22.09.2003 JP 2003330569
(71) Applicant: RIKEN, Wako-shi, Saitama 351-0198 (JP)
(72) Inventor: DEMURA, Taku, Yokohama-shi, Kanagawa 2300074 (JP); FUKUDA, Hiroo, Tokyo 1350044 (JP)
(74) Representative: Woods, Geoffrey Corlett
(86) International application number: PCT/JP2004/014308
(87) International publication number: WO 2005/028646

(57) **Abstract**

Conventional techniques for preparing a chimera gene having an inverted repeat sequence of a target sequence suffer from complications since a target sequence is inserted into 2 sites on a vector in sense and antisense orientations, and restriction enzyme recognition sequences must be independently provided at an insertion site of a vector and both ends of the target sequence.

In this invention, a cassette construct comprising an arbitrary adaptor sequence and an inverted adaptor sequence separated by an arbitrary spacer sequence or a plasmid vector comprising such cassette construct incorporated therein is prepared, a target sequence is ligated to either or both ends of the cassette construct, or a target sequence is inserted into one end of the cassette construct on the plasmid vector, followed by PCR. Thus, a chimera gene having an inverted repeat sequence is prepared.

SEQUENCE LISTING

<110> RIKEN

<120> Method of preparing inverted repeat sequence

<130> PH-2258-PCT

<150> JP 2003/330569
<151> 2003-09-22

<160> 5

<170> PatentIn version 3.1

<210> 1
<211> 53
<212> DNA
<213> artificial

<220>
<223> adaptor sequence

<400> 1
gagatattac ctgcaggtac tcacccgggt gagtacctgc aggtaatatc tca 53

<210> 2
<211> 33
<212> DNA

<213>  artificial

<220>

<223>  Primer A

<400>  2
cccctcgagg tcgacggtat cgataagctt gat                                    33

<210>  3
<211>  35
<212>  DNA
<213>  artificial

<220>

<223>  Primer B

<400>  3
gattgagata ttacctgcag gtactcaccc gggtg                                  35

<210>  4
<211>  1144
<212>  DNA
<213>  artificial

<220>

<223>  DNA sequence containing the first intron of the Arabidopsis thaliana FAD2 gene

<400>  4
atccaggtcc gtcgcttctc ttccatttct tctcattttc gattttgatt cttatttctt        60

tccagtagct cctgctctgt gaatttctcc gctcacgata gatctgctta tactccttac 120

attcaacctt agatctggtc tcgattctct gtttctctgt tttttctttt tggtcgagaa 180

tctgatgttt gtttatgttc tgtcaccatt aataataatg aactctctca ttcatacaat 240

gattagtttc tctcgtctac aaaacgatat gttgcatttt cacttttctt ctttttttct 300

aagatgattt gctttgacca atttgtttag atctttattc tattttattt tctggtgggt 360

tggtggaaat tgaaaaaaaa aaaacagcat aaattgttat ttgttaatgt attcattttt 420

tggctatttg ttctgggtaa aaatctgctt ctactattga atctttcctg gattttttac 480

tcctattggg tttttatagt aaaaatacat aataaaagga aaacaaaagt tttatagatt 540

ctcttaaacc ccttacgata aaagttggaa tcaaaataat tcaggatcag atgctctttg 600

attgattcag atgcgattac agttgcatgg caaattttct agatccgtcg tcacatttta 660

ttttctgttt aaatatctaa atctgatata tgatgtcgac aaattctggt ggcttataca 720

tcacttcaac tgttttcttt tggctttgtt tgtcaacttg gttttcaata cgatttgtga 780

tttcgatcgc tgaattttta atacaagcaa actgatgtta accacaagca agagatgtga 840

cctgccttat taacatcgta ttacttacta ctagtcgtat tctcaacgca atcgtttttg 900

tatttctcac attatgccgc ttctctactc tttattcctt ttggtccacg cattttctat 960

ttgtggcaat ccctttcaca acctgatttc ccactttgga tcatttgtct gaagactctc 1020

ttgaatcgtt accacttgtt tcttgtgcat gctctgtttt ttagaattaa tgataaaact 1080

attccatagt cttgagtttt cagcttgttg attcttttgc ttttggtttt ctgcagaaac 1140

accc 1144

<210> 5

<211> 90

<212> DNA

<213> artificial

<220>

<223> Partial DNA sequence of the Zinnia elegans cDNA, Z8755

<400> 5

acacgtttcc tggagctgta acttgcatcg tgtatgcata ctctggagga atgtaaccaa 60
aggagcccgc aacagcgcta atactagcag 90

## Description

### Technical Field

The present invention relates to an RNA interference technique that can be used as a means for analyzing gene functions and that can be used for preparing functionally modified mutant animals or plants.

The present invention further provides a method for efficiently preparing a chimera gene having an inverted repeat sequence of a DNA fragment of a target gene that is used for RNA interference and a cassette construct therefor.

### Background Art

The antisense method (Non-Patent Document 1) and the sense method (Non-Patent Document 2) have been heretofore known as techniques for post-transcriptional regulation of gene expression. However, both of these techniques are insufficient in terms of efficiency of gene regulation in transformants.

In recent years, double-stranded RNA (dsRNA) has been found to be more effective in regulation of lowering of post-transcriptional gene expression than antisense or sense RNA in *Caenorhabditis elegans.* This phenomenon is referred to as "RNAi." RNAi starts from degradation of dsRNA into small fragments referred to as small interfering RNAs or siRNAs mediated by a Dicer enzyme, which is a member of the RNase III nuclease family.

Upon introduction of a very small amount of dsRNA into mammalian cells, interferon is generated, and apoptosis is induced. Accordingly, a method that would not induce apoptosis was studied. It was discovered that siRNA, which is short dsRNA generated upon Dicer-induced degradation of dsRNA, would not induce apoptosis and that it would be capable of post-transcriptional regulation. Further, several *Caenorhabditis elegans* genes were found to encode hairpin RNA and to regulate other genes. Based on such findings, whether or not hairpin-folded small RNA could inhibit functions of a given gene instead of siRNA was examined. As a result, short hairpin RNA (shRNA) was found to be capable of regulating gene expression at the same level as siRNA.

As a method for further efficiently suppressing post-transcriptional gene expression, a method involving the use of a chimera gene having an inverted repeat sequence of a DNA fragment of a target gene is known (Non-Patent Document 3).

A method, wherein a chimera gene having an inverted repeat sequence of a DNA fragment of a target gene is stably introduced into an organism, and dsRNA generated upon such introduction in the organism induces degradation of endogenous RNA in a sequence-specific manner (i.e., RNA interference), has been extensively used as a means for effectively analyzing gene functions.

A technique wherein homologous gene recombination is applied to the insertion of a DNA fragment of a target gene has been reported (Patent Document 1; Non-Patent Document 4).

Patent Document 1: U.S. Patent Publication No. 20030049835
Non-Patent Document 1: Proc. Natl. Acad. Sci. U.S.A., Vol. 83, 5372-5376 (cited in Non-Patent Document 3)
Non-Patent Document 2: Plant Cell, Vol. 2, p. 291 (cited in Non-Patent Document 3)
Non-Patent Document 3: The Plant Journal, Vol. 33, pp. 793-800
Non-Patent Document 4: Plant J., 27, 581-590, 2001

### Disclosure of the Invention

Conventional techniques for constructing a chimera gene having an inverted repeat sequence of a DNA fragment of a target gene (a target sequence) require separate insertion of the target sequence to 2 sites on an arbitrary vector, and usually on a plasmid vector, in sense and antisense orientations or vice versa.

In general, such target sequence insertion is carried out via cleavage of DNA with a restriction enzyme and via ligase-mediated binding. In order to accurately insert the target sequence in sense and antisense orientations or vice versa, restriction enzyme recognition sequences must be independently present at 2 target-sequence-insertion sites on the vector and the target sequence must have restriction enzyme recognition sequences corresponding to the target-sequence-insertion sites.

Construction of a chimera gene having an inverted repeat sequence of a target sequence may be occasionally difficult via standard procedures, depending on the type of nucleotide sequence of the target sequence. Thus, this technique is not suitable for extensive analysis of gene functions.

Also, a technique wherein homologous gene recombination is applied to target sequence insertion has been reported (Wesley et al., Plant J., 27, 581-590, 2001; U.S. Patent Publication No. 20030049835). In this technique, a pair of recognition sequences for an enzyme used for homologous gene recombination must be provided at both ends of the target sequence, and it is necessary to carry out PCR involving the use of an oligonucleotide primer specific to the nucleotide sequences at both ends of the target sequence or to insert a target sequence between a pair of recognition sequences of the enzyme used for homologous gene recombination on a vector. In order to construct a chimera gene having an inverted repeat sequence with the use of a target sequence comprising the aforementioned enzymes used for homologous gene recognition sequence, each target sequence must be independently subjected to homologous gene recombination.

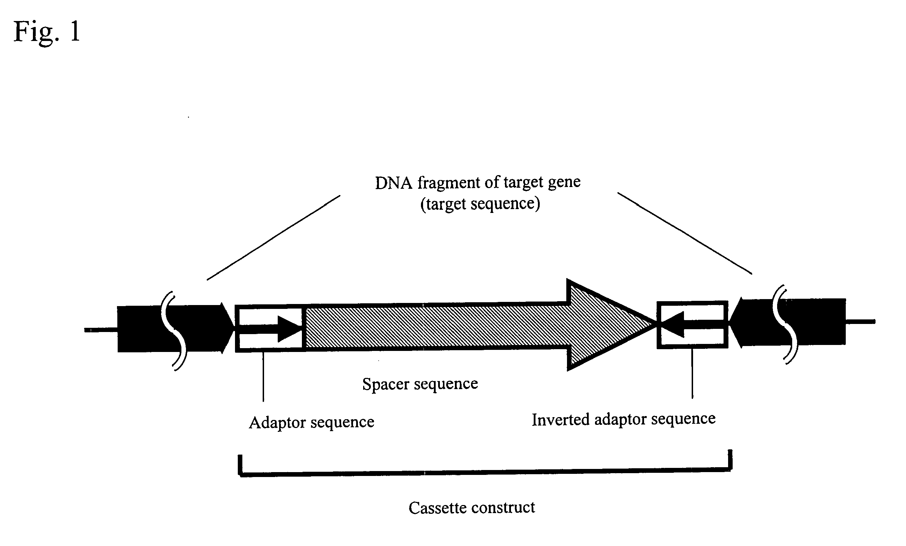

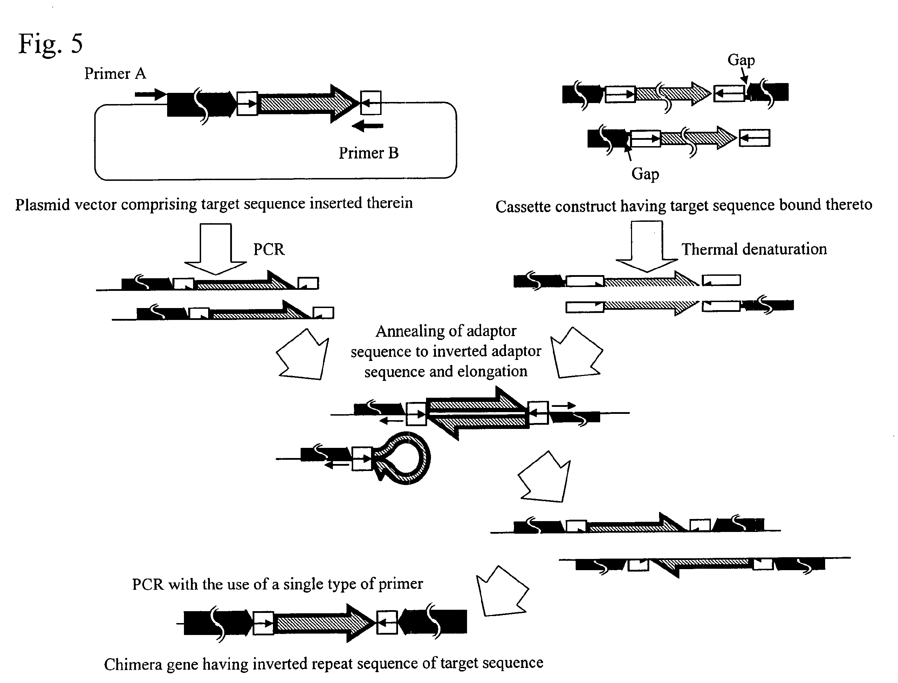

At the outset, a cassette construct, which is a DNA construct comprising an arbitrary nucleotide sequence (an adaptor sequence) and an inverted repeat nucleotide sequence of the adaptor sequence (an inverted adaptor sequence) separated by an arbitrary spacer sequence or a plasmid vector having such cassette construct incorporated therein is prepared. Subsequently, a DNA fragment of the target gene (the target sequence) is bound to either or both ends of the cassette construct. Alternatively, the target sequence is inserted into a site outside either end of the cassette construct on the plasmid vector. Further, the cassette construct comprising the target sequence bound thereto is used as a template to conduct PCR with the use of a single type of primer corresponding to either end of the target sequence. Alternatively, the plasmid vector having the target sequence inserted therein is used as a template to conduct PCR with the use of a primer (primer A) corresponding to a nucleotide sequence of a plasmid vector located outside of the aforementioned target-sequence-insertion site and a primer (primer B) corresponding to a nucleotide sequence comprising an adaptor sequence or an inverted adaptor sequence located on the side of the cassette construct opposite from the target-sequence-insertion site and part of a spacer sequence. This can yield a double-stranded PCR product, i.e., a chimera gene that contains an inverted repeat sequence of the target sequence via a lariat configuration resulting from temporary annealing between the adaptor sequence and the inverted adaptor sequence in a single single-stranded PCR product generated upon PCR or via a dimmer configuration resulting from temporary annealing between these adaptor sequences of 2 single-stranded PCR products, followed by elongation and PCR (Fig. 1 to Fig. 5).

In the present invention, a cassette construct, which is a DNA construct comprising an arbitrary nucleotide sequence (an adaptor sequence) and an inverted repeat nucleotide sequence of the adaptor sequence (an inverted adaptor sequence) separated by an arbitrary spacer sequence or a plasmid vector having such cassette construct incorporated therein is first prepared. Thus, a chimera gene having an inverted repeat sequence of a target sequence as shown in Fig. 1 can be efficiently prepared in the same manner, regardless of the type of nucleotide sequence of the target sequence.

When a large quantity of various genes are knocked out via the RNAi method, it is required that chimera genes having inverted repeat sequences of a target sequence can be easily prepared in the same manner. The present invention sufficiently satisfies such requirement.

This description includes part or all of the contents as disclosed in the description and/or drawings of Japanese Patent Application No. 2003-330569, which is a priority document of the present application.

### Brief Description of the Drawings

Fig. 1 schematically shows an inverted repeat sequence of a target sequence prepared in the present invention.
Fig. 2 shows a procedure 1 for preparing a cassette construct.
Fig. 3 shows a procedure 2 for preparing a cassette construct.
Fig. 4 shows a procedure for preparing a plasmid vector for preparing an inverted repeat sequence of a target sequence comprising a cassette construct incorporated therein.
Fig. 5 shows a procedure for preparing a chimera gene having an inverted repeat sequence of a target sequence.
Fig. 6 illustrates a cassette construct 1 for facilitating target sequence binding.
Fig. 7 illustrates a cassette construct 2 for facilitating target sequence binding.
Fig. 8 illustrates a cassette construct 3 for facilitating target sequence binding.
Fig. 9 illustrates a cassette construct 4 for facilitating target sequence binding.
Fig. 10 illustrates a cassette construct 5 for facilitating target sequence binding.
Fig. 11 illustrates a cassette construct 6 for facilitating target sequence binding.
Fig. 12 shows an embodiment of modification of a plasmid vector for preparing an inverted repeat sequence of a target sequence comprising a cassette construct incorporated therein.
Fig. 13 shows a method for preparing pGEMA and pGEMAS.
Fig. 14 shows a method for preparing pRNAi.
Fig. 15 shows a procedure for inserting the target sequence Z8755 into pRNAi.
Fig. 16 shows a procedure for preparing a chimera gene having an inverted repeat sequence of the target sequence Z8755.

### Preferred Embodiments of the Invention

### 1. Cassette construct

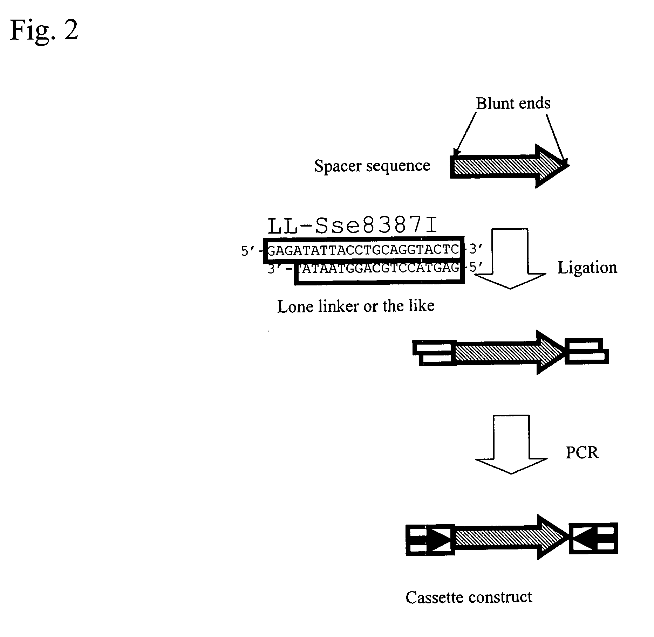

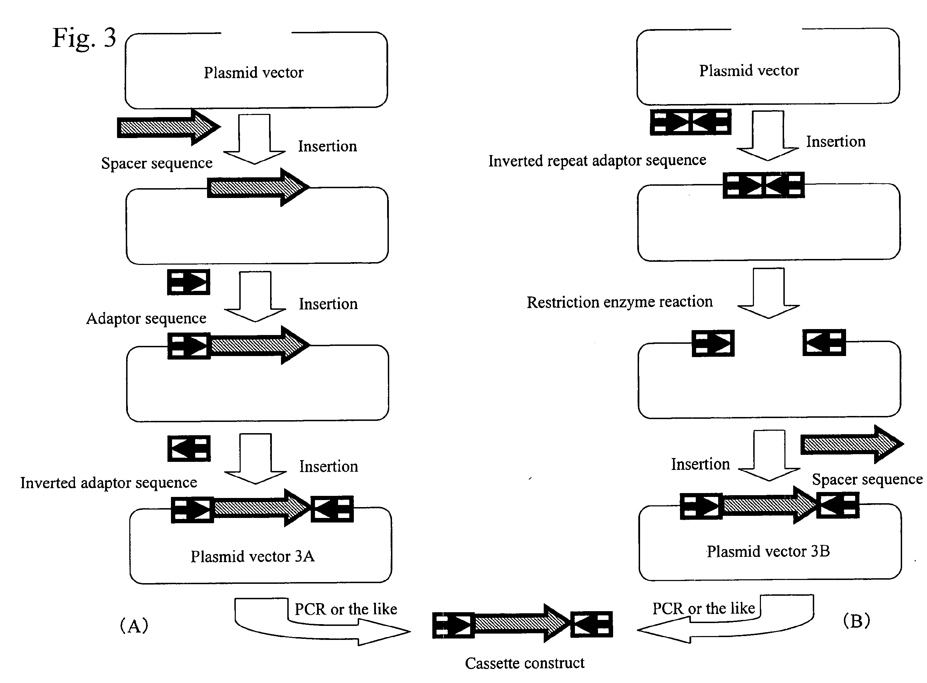

The present invention includes a DNA construct (a cassette construct) wherein an arbitrary sequence (a spacer or intervening sequence) is located between another arbitary nucleotide sequence (an adaptor sequence) and an inverted repeat nucleotide sequence of the adaptor sequence (an inverted adaptor sequence) (Figs. 2 and 3).

### 1-1. Spacer sequence (intron)

A spacer sequence must be a nucleotide sequence that is not related to a target sequence or an adaptor sequence (i.e., a sequence that does not complementarily bind to a target sequence or an adaptor sequence *in vitro* or *in vivo).* For example, an arbitrary sequence that does not complementarily hybridize to a target sequence or an adaptor sequence at the time of asymmetric PCR for chimera gene amplification can be used as a spacer sequence. A spacer sequence is preferably composed of 10 to 10,000 nucleotides, and more preferably 50 to 2,000 nucleotides, so that inverted repeat adaptor sequences can easily complementarily anneal to each other in a single molecule in the state of single-stranded DNA or RNA and they can be easily amplified in *E. coli.* Specific examples of spacer sequences that can be used include a partial fragment of a green fluorescent protein (GFP) from *Aequorea coerulescens* or that of a β-glucuronidase (GUS) structural gene from *E. coli.* More preferable examples include intron sequences, such as an intron sequence from the fatty acid desaturase (FAD2) gene from *Arabidopsis thaliana* or that from the ABC transporter gene of a fruit fly, so that a spacer sequence can be removed via splicing in the body of a plant, fruit fly, or the like.

### 1-2. Adaptor sequence

An adaptor sequence and an inverted adaptor sequence are each independently a nucleotide sequence that is not related to the nucleotide sequence of a target sequence, a spacer sequence, or a plasmid vector used (i.e., a sequence that does not complementarily bind to a target sequence, a spacer sequence, or a plasmid vector used *in vitro* or *in vivo).* Use of a sequence that enables annealing of an adaptor sequence to an inverted adaptor sequence under general PCR annealing conditions is preferable. Specifically, such sequence is designed to have 18 to 28 nucleotides, GC content of 50% to 60%, and Tm of 55°C to 80°C, in general. More specifically, a lone linker sequence reported by Ko et al. can be used as an adaptor sequence and a complementary sequence thereof can be used as an inverted adaptor sequence (Ko, M. S. H. et al., Nucleic Acids Res., 18, pp. 4293-4294, 1990).

An inverted adaptor sequence is not necessarily a completely inverted sequence of an adaptor sequence. However, it is necessary that such inverted adaptor sequence be able to complementarily hybridize to the target adaptor sequence at the time of asymmetric PCR for chimera gene amplification.

### 2-1. Preparation of cassette construct

The cassette construct can be prepared via conventional techniques. Preferably, a spacer sequence having blunt ends is first prepared, as shown in Fig. 2. Further, linker sequences each having a noncohesive protruding end and a blunt end, which are referred to as "lone linkers," can be ligated to the both blunt ends of the spacer sequence as an adaptor sequence and as an inverted adaptor sequence. Examples of lone linkers include LL-Sse8387I, such as LL-Sse8387IA (5'-GAGATATTACCTGCAGGTACTC-3') and LL-Sse8387IB (5'-GAGTACCTGCAGGTAATAT-3') and LL-SalI, such as LL-SalIA (5'-ATTGACGTCGACTATCCAGG-3') and LL-SalIB (5'-CCTGGATAGTCGACGTC-3') (Ko, M. S. H. et al., Nucleic Acids Res., 18, pp. 4293-4294, 1990). LL-Sse8387IA,B or LL-SAlIA,B is phosphorylated and annealed. The annealed LL-Sse8387I or LL-SalI is ligated to a spacer sequence having blunt ends. The thus prepared cassette construct can be amplified via PCR using, for example, LL-Sse8387I or LL-SalIA as a primer.

Alternatively, the cassette construct can be prepared in the following manner. A cassette construct is prepared on a plasmid vector via conventional techniques, and a portion of the cassette construct can be cleaved via restriction enzyme reaction or can be amplified via PCR. A cassette construct can be prepared on a plasmid vector by, for example, inserting an adaptor sequence and an inverted adaptor sequence at both ends of the spacer sequence on the plasmid vector comprising the spacer sequence inserted therein via a conventional technique, i.e., restriction enzyme reaction, followed by ligation, as shown in Fig. 3A. Alternatively, an oligonucleotide having an adaptor sequence and an inverted adaptor sequence adjacent to each other and a recognition sequence for an arbitrary restriction enzyme between these adaptor sequences and an oligonucleotide consisting of a complementary strand of the former oligonucleotide are prepared as shown in Fig. 3B. These oligonucleotides are annealed to each other to prepare an inverted repeat adaptor sequence, the resultant is incorporated into an arbitrary plasmid vector, and a spacer sequence can be then inserted in the middle of the inverted repeat adaptor sequence with the use of the arbitrary restriction enzymes. Restriction enzymes, the recognition sites for which do not exist on an adaptor sequence, a plasmid vector, and a spacer sequence can be used as the aforementioned restriction enzymes. Preferably, restriction enzymes that generate cohesive ends with both ends of the spacer sequence to be inserted can be used. A plasmid vector for TA cloning, such as the pGEM-T Easy Vector (Promega), can be preferably used as the plasmid vector with the addition of a single nucleotide, i.e., adenine (A), to the 3' end of the oligonucleotide.

### 2-2. Preparation of a plasmid vector for preparing an inverted repeat sequence of a target sequence comprising a cassette construct incorporated therein

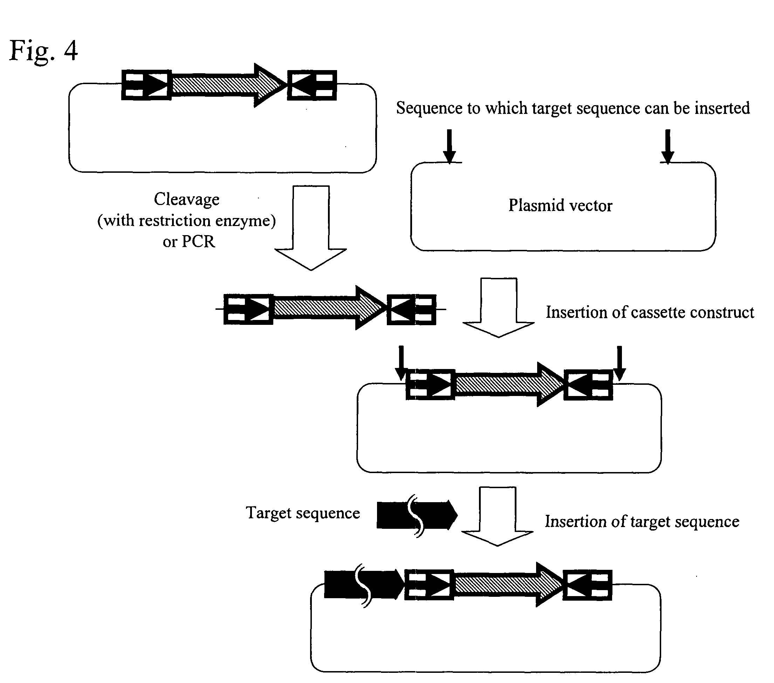

A plasmid vector for preparing an inverted repeat sequence of a target sequence comprising a cassette construct incorporated therein can be prepared via conventional techniques. For example, a restriction enzyme recognition site at which a target sequence can be inserted into the plasmid vector located adjacent to the cassette construct comprised in the plasmid vector 3A and the plasmid vector 3B as shown in Fig. 3 may be provided, and the resultant can be used as a plasmid vector for preparing an inverted repeat sequence of the target sequence having the cassette construct incorporated therein. Alternatively, a restriction enzyme recognition site that generates blunt ends on a plasmid vector adjacent to the cassette construct upon incorporation of the cassette construct prepared in 2-1 into an arbitrary plasmid vector may be provided as shown in Fig. 4. Thus, a plasmid vector for preparing an inverted repeat sequence of a target sequence that can easily insert a target sequence can be designed.

The plasmid vector having the cassette construct incorporated therein can be used for preparing the pool of chimera genes having inverted repeat sequences of a variety of gene DNA fragments based on cDNA of arbitrary animals or plants, and preferably based on homogenized cDNA.

### 2-3. Binding of a target sequence to a cassette construct and insertion of a target sequence into a plasmid vector for preparing an inverted repeat sequence comprising a cassette construct incorporated therein

A target sequence can be selected in accordance with the Patent Document 1. For example, a DNA fragment of the gene, the phenotype expression of which is to be suppressed, can be used as a target sequence. A target sequence is composed of 10 to a number of nucleotides required for sufficiently suppressing the phenotype expression of the target sequence, and such number is preferably at least 19 nucleotides, at least 21 nucleotides, at least 25 nucleotides, at least about 50 nucleotides, at least about 100 nucleotides, at least about 150 nucleotides, at least about 200 nucleotides, or at least about 500 nucleotides. A target sequence may be composed of about 1,000 nucleotides, if possible.

A target sequence is bound to either or both ends of a cassette construct. In such a case, use of a target sequence having unphosphorylated blunt ends is preferable. Such target sequence can be prepared via PCR with the use of a set of primers having the dephosphorylated 5' end and thermostable DNA polymerase that does not add (dA) to the 3' end or via PCR with the use of common thermostable polymerase, followed by removal of a (dA) protrusion at the 3' end via conventional techniques and dephosphorylation of PCR products. Alternatively, PCR is carried out with the use of a set of primers having the phosphorylated 5' end and thermostable DNA polymerase that does not add (dA) to the 3' end, followed by dephosphorylation of the 5' end of PCR products. Thus, a target sequence of interest can be prepared.

A target sequence is inserted into a site outside of an adaptor sequence or an inverted adaptor sequence on a plasmid vector for preparing an inverted repeat sequence of the target sequence, which comprises the cassette construct incorporated therein. Preferably, a DNA fragment of the target sequence is inserted into a site outside one end of the cassette construct with the use of a restriction enzyme, and the recognition sequence for which is present at a site outside one end of the cassette construct on the plasmid vector only. More preferably, a target gene is inserted into a site outside either end of the cassette construct, where it is proximal to the cassette construct. When a recognition sequence of the blunt-end-generating restriction enzyme is located outside the adaptor sequence, for example, the plasmid vector is cleaved with such restriction enzyme, and the cleavage product can be ligated to a blunt-ended target sequence with the aid of a ligase. When another restriction enzyme recognition sequence is present outside the adaptor sequence, a plasmid vector and the target sequence are treated with the same restriction enzyme, and they are allowed to bind to each other at their cohesive ends.

### 2-4. Modification of a cassette construct (pretreatment for binding a target sequence to either or both ends of a cassette construct)

In order to facilitate the binding of the target sequence to the cassette construct and the preparation of an inverted repeat sequence of the target sequence via PCR using the cassette construct having the target sequence bound thereto as a template, either or both ends of the cassette construct can be modified as a pretreatment.

Cassette constructs for facilitating target sequence binding are as described below.

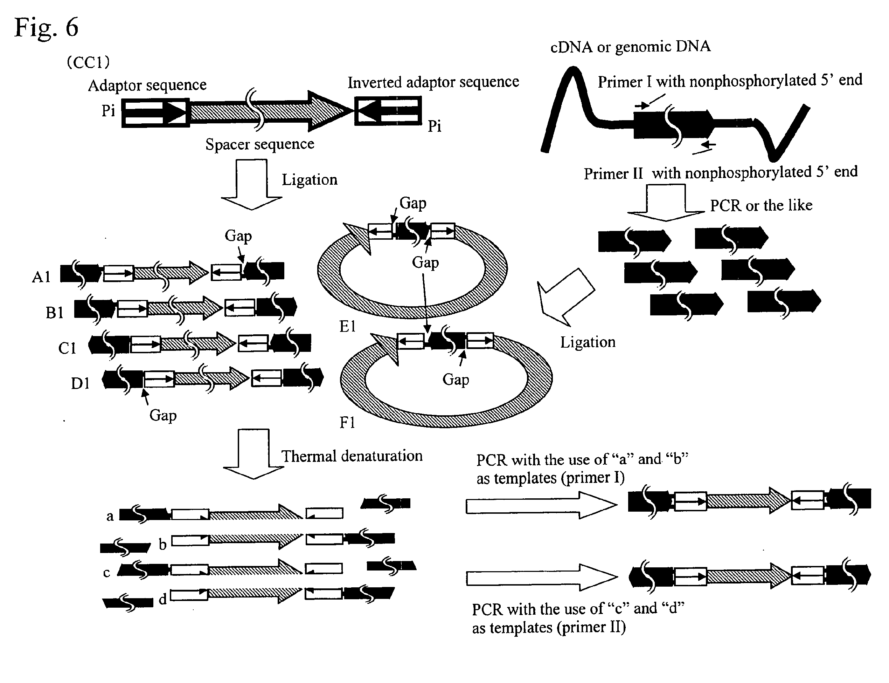

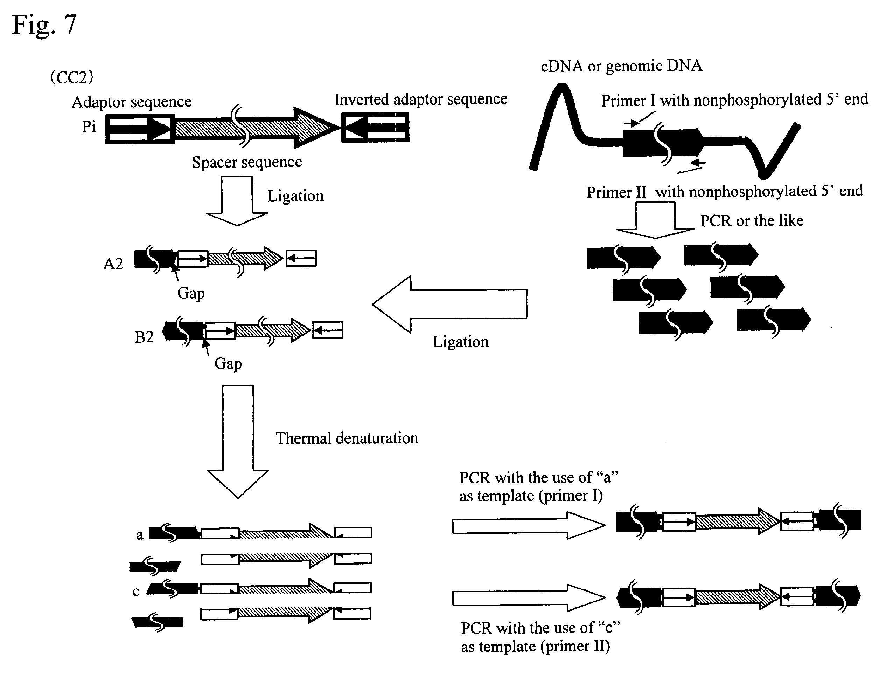

The cassette construct 1 for facilitating target sequence binding shown in Fig. 6 (hereafter abbreviated to "CC1") is designed to facilitate the binding of the target sequence, both ends of which are dephosphorylated, via phosphorylation of both 5' ends. CC1 can be easily prepared with the use of thermostable DNA polymerase that does not add (dA) to the 3' end, such as KOD-Plus- (Toyobo). The cassette construct 2 for facilitating target sequence binding shown in Fig. 7 (CC2) is designed to facilitate the binding of the target sequence, both ends of which are dephosphorylated, to a single end of the cassette construct via phosphorylation of a single end.

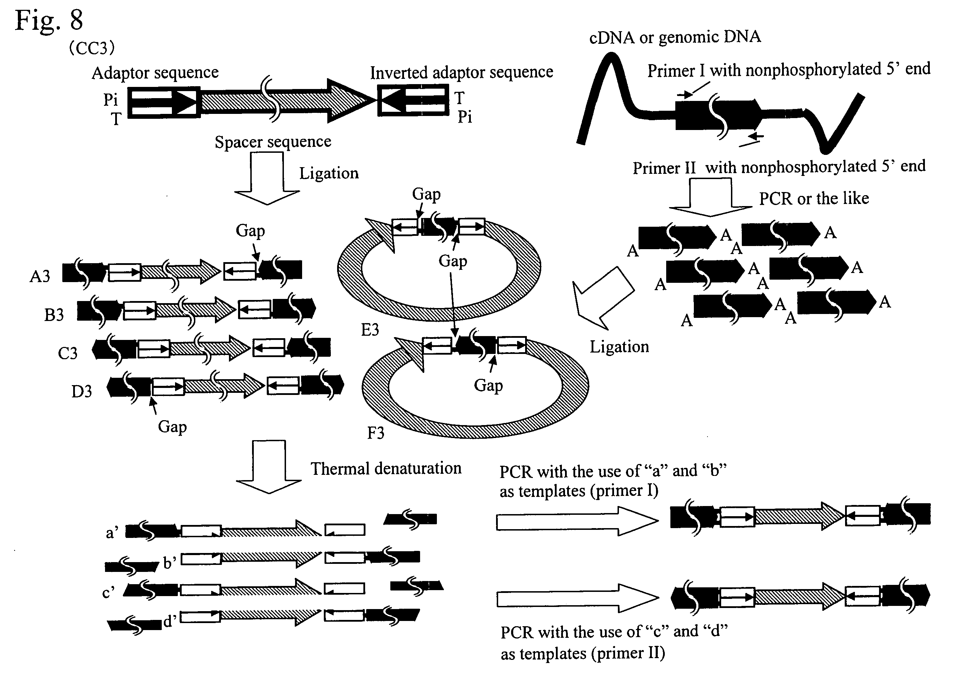

The cassette construct 3 for facilitating target sequence binding shown in Fig. 8 (CC3) is designed to facilitate the binding of the target sequence, which is amplified with the use of common thermostable DNA polymerase that adds (dA) to the 3' end and both ends of which are dephosphorylated, to the 3' end of the cassette construct via addition of (dT) to both 3' ends of CC1.

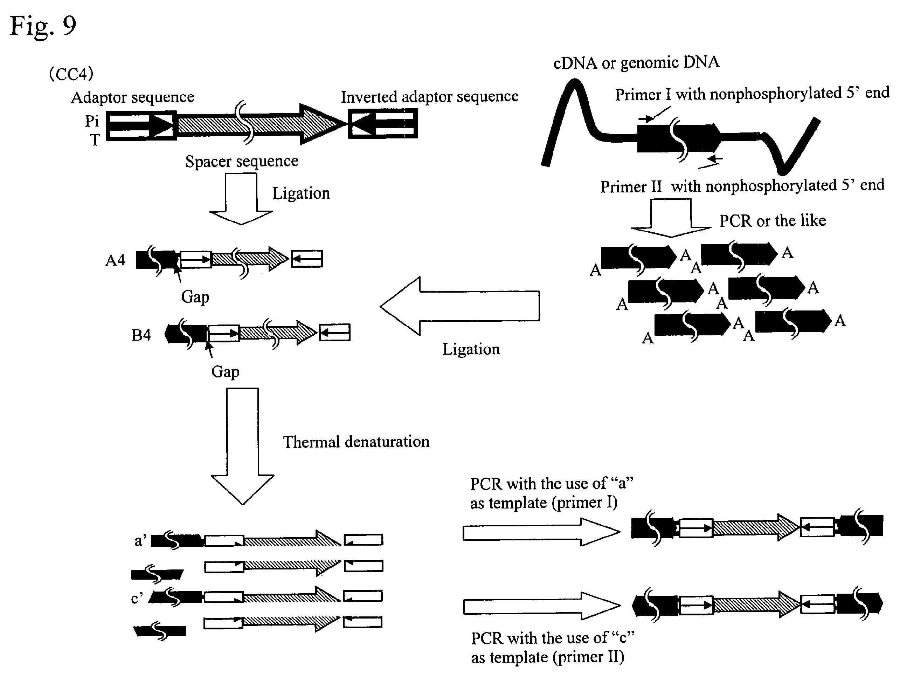

The cassette construct 4 for facilitating target sequence binding shown in Fig. 9 (CC4) is designed to facilitate the binding of the target sequence, which is amplified with the use of common thermostable DNA polymerase that adds (dA) to the 3' end and both ends of which are dephosphorylated, to a single 3' end of the cassette construct via addition of (dT) and phosphorylation of such single 3' end.

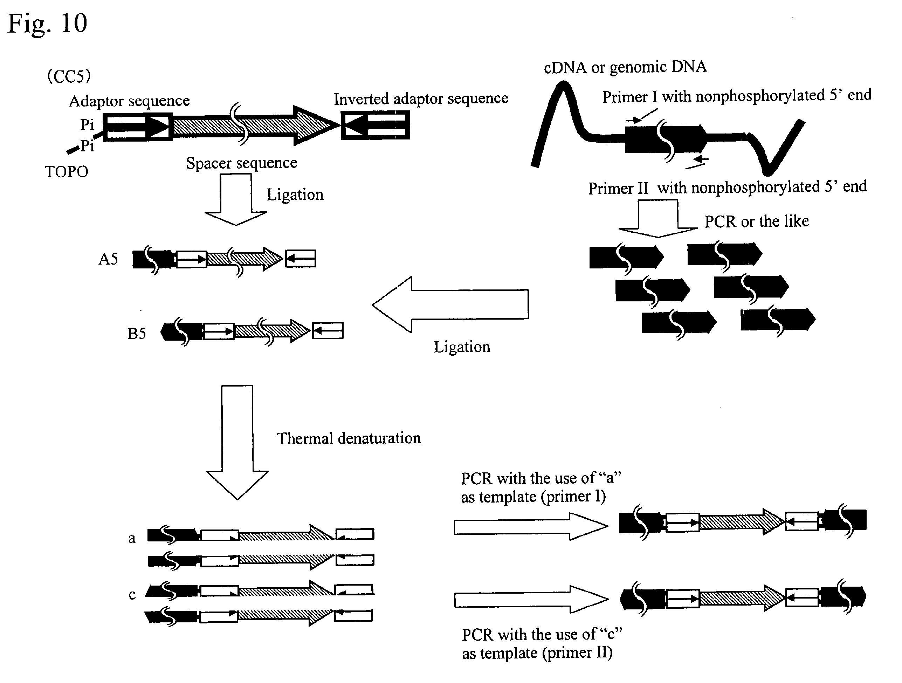

The cassette construct 5 for facilitating target sequence binding shown in Fig. 10 (CC5) is designed to facilitate the binding of the target sequence to a single end of the cassette construct via addition of topoisomerase I (Invitrogen) and phosphorylation of a single 3' end.

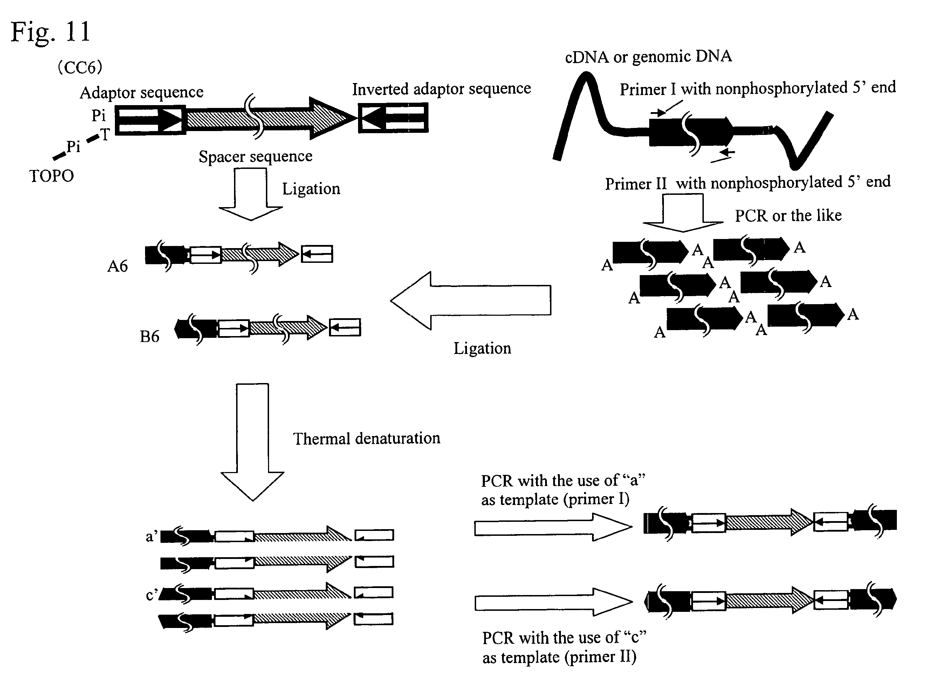

The cassette construct 6 for facilitating target sequence binding shown in Fig. 11 (CC6) is designed to facilitate the binding of the target sequence, which is amplified with the use of common thermostable DNA polymerase that adds (dA) to the 3' end and both ends of which are dephosphorylated, to a single end of the cassette construct via addition of (dT) and topoisomerase I to a single 3' end and phosphorylation of such single 3' end.

### 2-5. Modification of a plasmid vector for preparing an inverted repeat sequence of a target sequence comprising a cassette construct incorporated therein

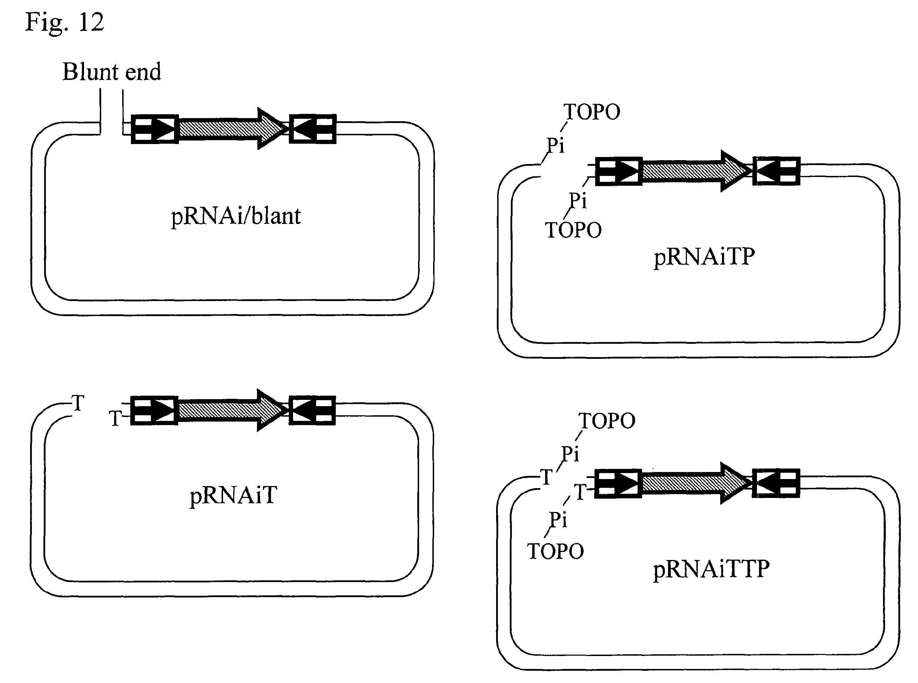

In order to facilitate the insertion of the target sequence into a plasmid vector for preparing an inverted repeat sequence of the target sequence comprising the cassette construct incorporated therein, the plasmid vector for preparing an inverted repeat sequence is cleaved with a restriction enzyme or the like, and the ends thereof can be processed so as to facilitate the insertion of the target sequence. More specifically, the cleaved ends are blunt-ended as with the case of pRN Ai/blunt shown in Fig. 12, (dT) is added to the cleaved 3' end as with the case of pRNAiT shown in Fig. 12, topoisomerase I is added to the cleaved 3' end as with the case of pRNAiTP shown in Fig. 12, or (dT) and topoisomerase I are added to the cleaved 3' end as with the case of pRNAiTTP shown in Fig. 12.

### 3. Amplification of chimera gene

A DNA product in which the target sequence has been bound to either or both ends of the cassette construct as shown in any of Fig. 6 to Fig. 11 is used as a template to conduct arbitrary gene amplification techniques, and preferably, PCR, with the use of a primer specific to one end of the target sequence (primer I or II). Thus, a chimera gene comprising a target sequence and an inverted repeat sequence thereof separated by the cassette construct can be prepared. Specifically, generation of a single-stranded DNA construct via thermal denaturation, PCR with the use of primer I or II may be carried out. Fig. 6 to Fig. 11 show the examples of PCR with the sole use of a primer specific to the target sequence, and different linkers (or adaptors) can be ligated to both ends of a cDNA or genomic DNA fragment via conventional techniques. In such a case, a primer set of the linker (or adaptor) portion can be used instead of a primer specific to the target sequence.

A plasmid vector comprising the target sequence inserted into a site located outside either end of the cassette construct is used as a template to conduct arbitrary gene amplification techniques, and preferably asymmetric PCR (in which the amount of a primer used is excessively large compared with another primer; asymmetric amplification, 4-1-2-6, "Amplification and detection of nucleic acid," Standard Techniques, (the Japanese Patent Office)). Thus, a chimera gene comprising the target sequence and an inverted repeat sequence separated by the cassette construct can be prepared. A set of the following primers is used: (1) a part of a plasmid vector located on the opposite side of the cassette construct from the inserted target sequence; and (2) an oligonucleotide designed to comprise an adaptor sequence or an inverted adaptor sequence located at a position farther from the target sequence and part of a spacer sequence adjacent thereto. When asymmetric PCR is carried out, it is preferable to use the primer (1) in amounts that are 5, 10, or approximately 100 times or more greater than that of the primer (2).

An arbitrary sequence composed of several nucleotides may be incorporated into the 5' side of the primer to facilitate the incorporation of the amplified chimera genes into various types of vectors.

The aforementioned asymmetric PCR results in amplification of excessive amounts of single-stranded PCR products composed of a target sequence, an adaptor sequence, a spacer sequence, and an inverted adaptor sequence ligated in that order. The adaptor sequence and part of the inverted adaptor sequence in the resulting single-stranded PCR product are temporarily annealed to each other in a single molecule to form a lariat configuration, or these sequences are annealed to each other between 2 molecules, followed by elongation and PCR amplification with the sole use of the primer (1). Thus, a chimera gene having an inverted repeat sequence can be obtained as a double-stranded PCR product.

Also, a chimera gene having a target sequence and an inverted repeat sequence thereof can be obtained as a double-stranded PCR product without asymmetric PCR. For example, general PCR (symmetric PCR) is carried out, a single-stranded PCR product elongated from the aforementioned primer (1), i.e., a single-stranded PCR product composed of a target sequence, an adaptor sequence, a spacer sequence, and an inverted adaptor sequence ligated in that order, is recovered, and the resultant is subjected to PCR with the sole use of the aforementioned primer (1). The adaptor sequence and part of the inverted adaptor sequence in the resulting single-stranded PCR product are temporarily annealed to each other in a single molecule to form a lariat configuration, or these sequences are annealed to each other between 2 molecules, followed by elongation and PCR amplification with the sole use of the primer (1). Thus, a chimera gene having an inverted repeat sequence can be obtained as a double-stranded PCR product.

### 4. Incorporation into expression vector and preparation of transformant

An amplification product comprising the inverted repeat sequence of the target sequence can be incorporated into an adequate expression vector.

The resulting expression vector can be introduced into a host cell, such as a plant cell.

Hereafter, the present invention is described in greater detail with reference to the examples, although the technical scope of the present invention is not limited thereto.

### Examples

### [Example 1]

A chimera gene shown in Fig. 1 was prepared in accordance with the following steps (1) to (3) as an example of preparation of a chimera gene having an inverted repeat sequence of a DNA fragment of a target gene (a target sequence) according to the present invention.

### (1) Preparation of pRNAi

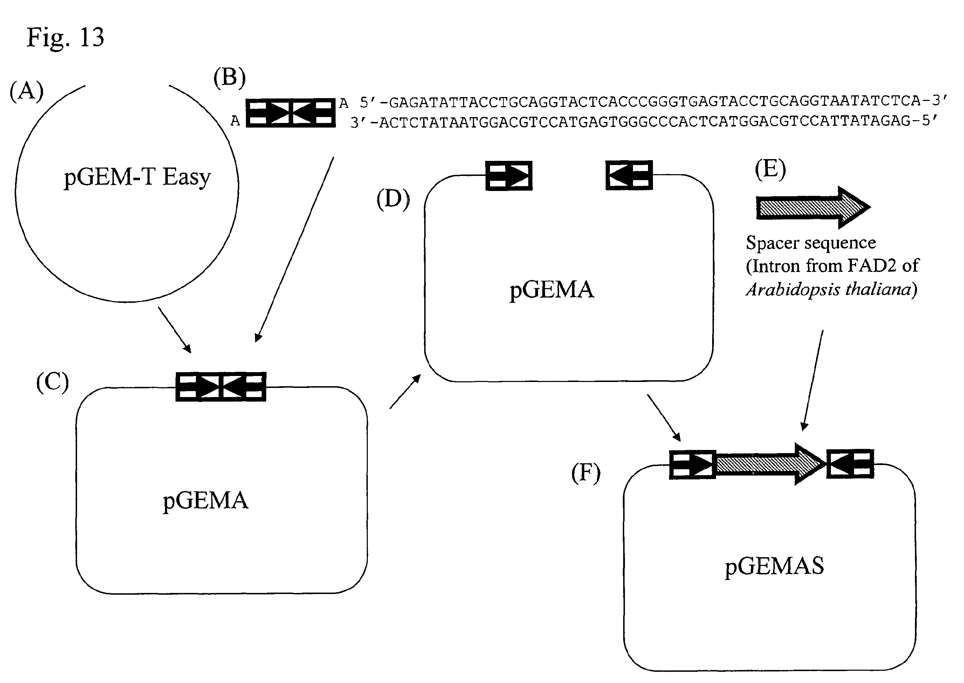

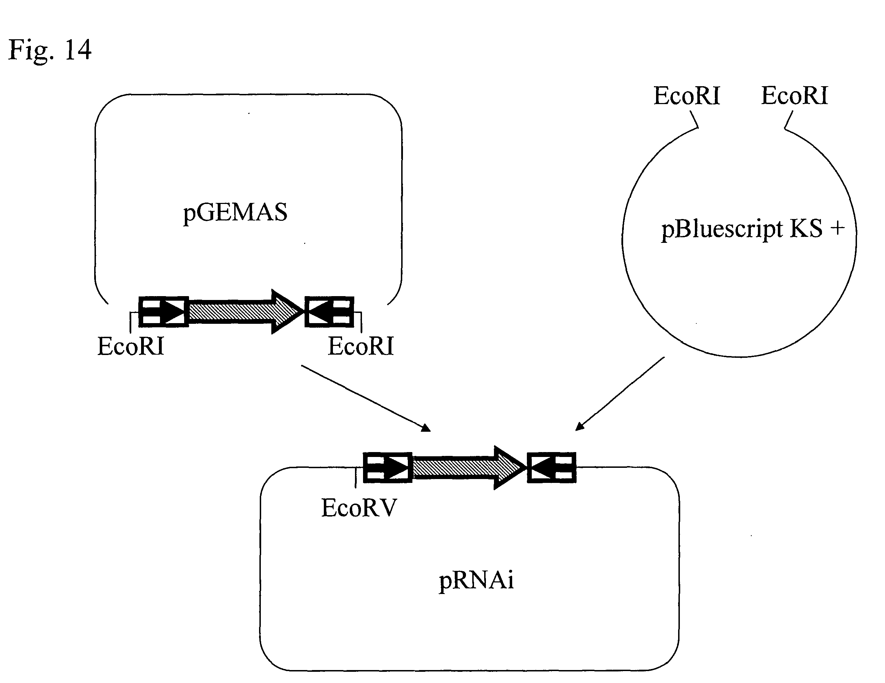

An oligonucleotide having an arbitrary adaptor sequence and an inverted arbitrary sequence between which a spacer sequence can be inserted in the center is synthesized. An oligonucleotide (5'-GAGATATTACCTGCAGGTACTCACCCGGGTGAGTACCTGCAGGTAATATCTC A-3') having a recognition sequence CCCGGG for the restriction enzyme *Sma*I in the center thereof and having A at its 3' end was synthesized herein, and this oligonucleotide was annealed (Fig. 13B), followed by cloning into a plasmid vector. Since protrusions (A) were generated at both 3' ends via annealing, the resultant was cloned into a commercially available TA cloning vector (the pGEM-T Easy Vector, Promega) (Fig. 13A) to prepare pGEMA (Fig. 13C). Further, a spacer sequence was inserted into the center of the cloned inverted repeat sequence (Fig. 13D to 13F). The center portion of the inverted repeat sequence of pGEMA was cleaved with the restriction enzyme *Sma*I (Fig. 13D), as a spacer sequence, a DNA fragment containing the intron sequence of the FAD2 genes from *Arabidopsis thaliana* was amplified via PCR (Fig. 13E, SEQ ID NO: 4), and the amplified fragment was inserted with the aid of T4 DNA ligase to prepare pGENAS (Fig. 13F). Subsequently, a region containing a cassette construct was cleaved with the restriction enzyme *Eco*RI, the recognition sequences of which are located at both sides of the cloning site of an oligonucleotide on the pGEM-T Easy Vector, and the cleavage product was inserted and ligated to the *Eco*RI cleavage site of the pBluescript II KS plasmid vector (Stratagene) with the use of T4 DNA ligase to prepare pRNAi comprising the recognition sequence for the restriction enzyme EcoRV at a position located outside of the adaptor sequence of the cassette construct (Fig. 14). Subsequently, the nucleotide sequence of pRNAi was examined via conventional techniques.

### (2) Insertion of DNA fragment of target gene (target sequence) into pRNAi

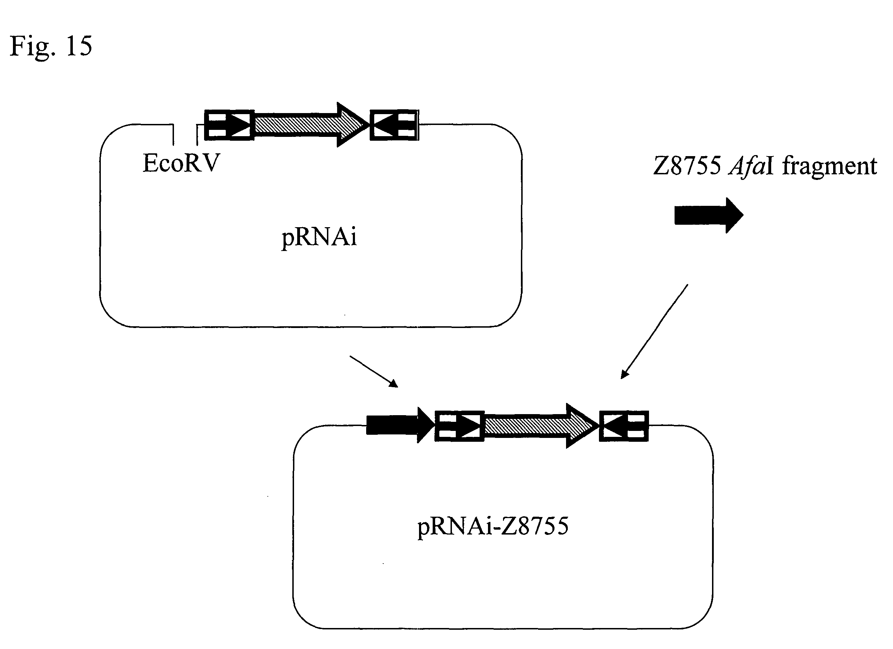

A target sequence was inserted into a site outside one end of the cassette construct on pRNAi. Part of cDNA of a receptor protein kinase gene of zinnia (Z8755, DDBJ Accession No. AU293996, T. Demura et al., PNAS, 99, 15794-15799), which had been cloned into the pGEM-T Easy Vector (Invitrogen), was inserted into a site outside of the adaptor sequence. At the outset, a set of primers corresponding to the sequence located outside of the cloning site on the vector; i.e., a forward primer (TGTAAAACGACGGCCAGT) and a reverse primer (CAGGAAACAGCTATGACC), were used to amplify via PCR a DNA fragment containing cDNA of Z8755, and a partial DNA fragment having blunt ends was prepared using the restriction enzyme *Afa*I (SEQ ID NO: 5). The resultant was inserted and ligated to the restriction enzyme *Eco*RV cleavage site of pRNAi with the aid of T4 DNA ligase (Fig. 15).

### (3) Preparation of a chimera gene having an inverted repeat sequence of a target sequence

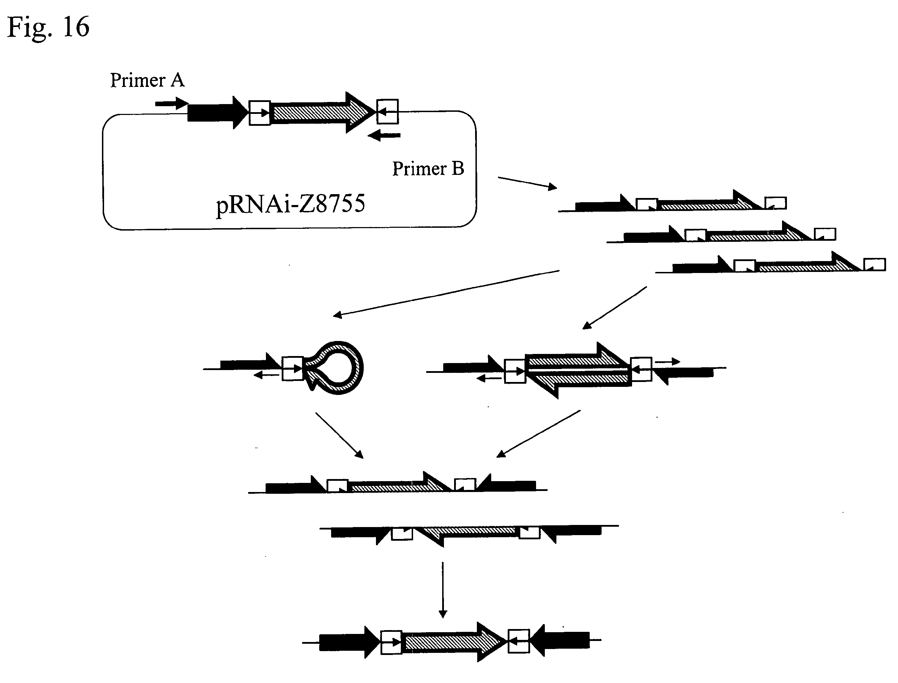

A plasmid comprising a target sequence inserted therein was used as a template to conduct PCR with the use of a primer (primer A) designed based on the nucleotide sequence of a vector located immediately outside of the target-sequence-insertion site and a primer (primer B) designed based on part of a spacer sequence and a nucleotide sequence containing an inverted repeat sequence located on the side of the cassette construct opposite from the DNA insertion site. In such a case, the amount of primer A used was excessively large compared with that of primer B. Thus, a double-stranded PCR product comprising a target sequence and an inverted repeat sequence thereof separated by a cassette construct was prepared. This is considered to be via a lariat configuration resulting from temporary annealing between the adaptor sequence and the inverted adaptor sequence in a single single-stranded PCR product generated upon PCR or via a dimmer configuration resulting from temporary annealing between these adaptor sequences of 2 single-stranded PCR products, followed by elongation and PCR. As shown in Fig. 16, oligonucleotide RNAiF (CACCCCTCGAGGTCGACGGTATCGATAAGCTTGAT) comprising a nucleotide sequence (CCCCTCGAGGTCGACGGTATCGATAAGCTTGAT) located immediately outside the recognition site for the restriction enzyme EcoRV of pBluescript II KS and having at its 5' end a sequence for cloning into pENTR/D Topo Vectpr (Invitrogen) was used at a final concentration of 0.6 µmol/l as primer A. Also, oligonucleotide RNAiR (GATTGAGATATTACCTGCAGGTACTCACCCGGGTG) synthesized from a complementary strand of the 3' end (CACCC) of the spacer sequence and the nucleotide sequence (GGGTGAGTACCTGCAGGTAATATCT) of an inverted adaptor sequence was used at a final concentration of 0.6 µmol/l as primer B. The reaction was carried out in 50 µl of a reaction solution comprising about 2 ng of a plasmid (pRNAi-Z8755) comprising target sequence (a partial sequence of Z8755), 1 µl of thermostable DNA polymerase (KOD-Plus-, TOYOBO), 5 µl of 10x buffer (TOYOBO), 2 µl of 25 mM MgSO₄ (TOYOBO), 5 µl of 2 mM dNTP (TOYOBO), 3 µl of 10 µM RNAiF, and 3 µl of 1 µM RNAiR, and thermal denaturation was carried out at 94°C for 2 minutes, followed by the reaction at 94°C for 15 seconds and at 68°C for 3 minutes and 30 seconds. Such PCR cycle was repeated 50 times.

### (4) Subcloning of a chimera gene having an inverted repeat sequence of a target sequence

The PCR product amplified via PCR in (3) above was subcloned into a plasmid vector via conventional techniques, and the nucleotide sequence thereof was determined to examine whether or not the target sequence had an inverted repeat sequence. The pENTR/D TOPO vector (Invitrogen) was used as a plasmid vector, and the nucleotide sequence was determined via cycle sequencing.

### Industrial Applicability

The present invention can be applied to technical fields where transformants and knock-out animals or plants are produced via an RNAi technique.

All publications, patents, and patent applications cited herein are incorporated herein by reference in their entirety.

### Sequence Listing Free Text

SEQ ID NO: 1 represents a ligation product of an adaptor sequence and an inverted sequence thereof. SEQ ID NOs: 2 and 3 represent primers. SEQ ID NO: 4 represents a DNA sequence containing an intron sequence of the FAD2 gene from *Arabidopsis thaliana.* SEQ ID NO: 5 represents a partial DNA sequence of zinnia cDNA (Z8755).

## Claims

1. A cassette construct for preparing an inverted repeat sequence of a target sequence consisting of an adaptor sequence, a spacer sequence, and an inverted sequence of the adaptor sequence.

2. The cassette construct according to claim 1, wherein the spacer sequence is an intron sequence.

3. The cassette construct according to claim 1 or 2, wherein either or both ends of the cassette construct are pretreated for target sequence binding.

4. The cassette construct according to any one of claims 1 to 3, which comprises a target sequence bound thereto at its either or both ends.

5. A method for preparing an amplification product comprising an inverted repeat sequence of a target sequence via PCR with the use of the cassette construct according to claim 4 as a template and a single primer derived from a sequence at either end of the target sequence.

6. A method for preparing an inverted repeat sequence of a target sequence via PCR with the use of the cassette construct according to claim 4 as a template.

7. A plasmid comprising the cassette construct according to claim 4 incorporated therein.

8. A method for preparing an inverted repeat sequence of a target sequence via PCR with the use of the plasmid according to claim 7 as a template.

9. The method for preparing an inverted repeat sequence of a target sequence according to claim 8, wherein PCR is asymmetric PCR.

10. The method according to claim 8 or 9, wherein a 3' end of a primer used in PCR contains a spacer sequence.

11. An expression vector comprising the inverted repeat sequence of the target sequence prepared by the method according to any one of claims 5, 6, 8, 9, and 10.

12. A host cell transformed with the expression vector according to claim 11.
